# EUROPÄISCHE PATENTSCHRIFT

(11) **EP 3 216 854 B1**
(45) Veröffentlichungstag und Bekanntmachung des Hinweises auf die Patenterteilung: **24.06.2020**
(21) Anmeldenummer: 17159468.2
(22) Anmeldetag: 06.03.2017
(51) Int. Cl.: C12M 1/107, C12M 1/00, C12M 1/34, C12M 1/36

(54) **VERFAHREN ZUM BETREIBEN EINER BIOGASANLAGE SOWIE BIOGASANLAGE**
METHOD FOR OPERATING A BIOGAS FACILITY AND BIOGAS FACILITY
PROCÉDÉ DE FONCTIONNEMENT D'UNE INSTALLATION DE BIOGAZ ET INSTALLATION DE BIOGAZ

(30) Priorität: 07.03.2016 DE 102016104070
(43) Veröffentlichungstag der Anmeldung: 13.09.2017
(73) Patentinhaber: E.ON Bioerdgas GmbH, 45131 Essen (DE)
(72) Erfinder: Wolf, Dieter, 46286 Dorsten (DE); Deupmann, Hermann, 48432 Rheine (DE)
(74) Vertreter: Harlacher, Mechthild

(56) Entgegenhaltungen:
- EP-A2- 2 528 192
- EP-A2- 2 586 868
- EP-A2- 2 832 846
- DE-A1-102008 063 250
- DE-A1-102009 018 126
- DE-A1-102011 051 135
- DE-B3-102014 113 413
- US-A1- 2012 065 788

## Beschreibung

Die Erfindung betrifft ein Verfahren zum Betreiben einer Biogasanlage, die mindestens einen Fermenter, mindestens einen Gasspeicher für das Biogas und mindestens einen Gasabnehmer aufweist, wobei dem Fermenter ein Substrat in einer vorgegebenen Gesamtmenge während eines vorgegebenen Zeitraums zum Vergären zugeführt wird und dem Gasabnehmer Biogas aus dem Gasspeicher zugeleitet wird. Ferner betrifft die Erfindung eine Biogasanlage.

Die Erzeugung von Biogas aus einem Substrat bzw. Biomasse aus biologisch abbaubaren Rohstoffen ist eine der wesentlichen Technologien der regenerativen Energieerzeugung. Das Substrat kann aus nachwachsenden Rohstoffen oder Abfällen bestehen. Bei der Zersetzung organischer Stoffe bildet sich Biogas als Stoffwechselprodukt von anaeorben Bakterien, insbesondere Methanbakterien, unter Ausschluss von Sauerstoff. Grundsätzlich gibt es zwei Arten von derartigen Biogasanlagen:
Bei den Biogasdirektverstromungsanlagen wird das in dem Fermenter oder den Fermentern entstehende Biogas bzw. Rohbiogas nach einer gewissen Reinigung in Gasmotoren bzw. Blockheizkraftwerken in Strom und Wärme umgewandelt.

Bei Biogaseinspeiseanlagen wird das in dem Fermenter oder den Fermentern entstehende Rohbiogas in einer nachgeschalteten Aufbereitungsanlage aufbereitet, insbesondere indem Kohlendioxid abgetrennt wird und anschließend ,falls erforderlich, mit Flüssiggas konditioniert, gereinigt, getrocknet und so verdichtet, dass es in das öffentliche Erdgasversorgungsnetz eingespeist werden kann. Beide Anlagentypen weisen mindestens einen Fermenter auf, in dem das zugeführte Substrat vergoren wird. Abhängig von den Fermentertypen und der Anlagengröße hat eine Anlage einen oder mehrere Fermenter.

Die Produktion von Biogas wird durch verschiedene Faktoren, wie Substratbeschaffenheit, Temperatur, Substratwechsel beeinflusst, so dass die Biogaserzeugung Schwankungen unterliegt. Zum Ausgleich der Schwankungen in der Biogaserzeugung oder der Biogasabnahme sind die Anlagen mit Gasspeichern ausgestattet.

Die Gasspeicher können mit den Fermentern eine Baueinheit bilden, insbesondere sich auf den Fermentern befinden oder als eigenständige Gasspeicher ausgebildet sein. Es handelt sich in der Regel um drucklose Speicher oder um Speicher mit einem sehr geringen Überdruck. Aus der Praxis sind Fermenter mit Gasspeichern bekannt, die als Doppelmembran-Tragluftspeicher ausgeführt sind. Der Tragluftdruck kann mittels eines Gebläses eingestellt werden. Dies ist bei Gasspeichern, die nur eine gummiartige Membran bzw. eine einlagige flexible Haube bzw. eine elastische oder bewegliche Behälterabdeckung aufweisen, nicht möglich. Wenn sich das Volumen in dem Gasspeicher vergrößert, dehnt sich die Haube aus. Derartige Hauben sind nur für geringe Überdrücke geeignet. Die meteorologischen Umgebungsbedingungen, insbesondere Wind und Sonneneinstrahlung haben einen Einfluss auf das gespeicherte Gasvolumen. Sonneneinstrahlung lässt die Gastemperatur im Speicher ansteigen, so dass sich die Haube ausdehnt. Die Hauben werden durch den Überdruck in den Fermentern mehr oder weniger aufgeblasen und können so abhängig von ihrer Größe ein bestimmtes Gasvolumen speichern. Daher sind die Hauben mit Überdrucksicherungen ausgestattet, die Rohbiogas aus den Fermenten in die Atmosphäre abströmen lassen, wenn ein eingestellter Druck überschritten wird. Wenn in einer Anlage mehr Biogas erzeugt wird, als bei den Direktverstromungsanlagen von den Gasmotoren in Strom und Wärme umgewandelt werden kann, wird das überschüssige Biogas Gasbrennern in Gaskesseln oder Fackeln zugeführt und dort verbrannt.

Für die Aufbereitung von Biogas zu Bioerdgas existieren verschiedene Technologien. Den gängigen Technologien wie Druckwasserwäsche, Aminwäsche, Selexolwäsche, Druckwechseladsorption und Membranverfahren ist gemein, dass die Aufbereitungskapazität begrenzt ist. Daher muss auch bei Biogaseinspeiseanlagen überschüssiges Rohbiogas, welches nicht in der Aufbereitungsanlage aufbereitet und in das öffentliche Erdgasversorgungsnetz eingespeist werden kann, ebenfalls abgefackelt oder abgeblasen werden.

EP 2 832 846 löst dieselbe Aufgabe durch ein Gebläse, welches Druckdifferenzen in zwei miteinander verbunden Gasspeichern ausgleicht und offenbart eine Biogasanlage mit mindestens zwei für einen Gastransfer durch Gasleitungen beliebig miteinander verbundenen Gasspeichern, deren Füllstände mittels Erfassungsvorrichtungen ermittelt und einer Regeleinrichtung zugeführt werden.

Vor dem oben beschriebenen Hintergrund liegt der Erfindung die Aufgabe zugrunde, ein Verfahren der eingangs genannten Art und eine Biogasanlage so zu verbessern, dass die Biogasproduktion und Speicherung optimiert wird, insbesondere dass die Erzeugung von energetisch nicht nutzbaren Biogasmengen vermieden wird.

Die gestellte Aufgabe wird bei einem Verfahren der eingangs genannten Art durch die Merkmale des Anspruchs 1 gelöst.

Das erfindungsgemäße Verfahren ist dadurch gekennzeichnet, dass in einer Regelungseinrichtung ein vorgegebener oberer Grenzwert und ein vorgegebener unterer Grenzwert für das Gasvolumen in dem Gasspeicher hinterlegt sind, dass während des Gärprozesses der Istwert des Gasvolumens in dem Gasspeicher messtechnisch erfasst und in der Regelungseinrichtung gespeichert wird, dass vor Beginn des vorgegebenen Zeitraums ein meteorologischer Datensatz in der Regelungseinrichtung hinterlegt wird und dass in Abhängigkeit von dem meteorologischen Datensatz und in Abhängigkeit von der Abweichung des gemessenen Istwertes vom oberen Grenzwert und/oder unteren Grenzwert die Substratzufuhr während des vorgegebenen Zeitraums gesteuert oder geregelt wird.

In Abhängigkeit von der Abweichung des Istwertes von dem Sollwert des Gasvolumens wird ein Steuersignal zur Steuerung der Substratzufuhr während des vorgegebenen Zeitraums gebildet.

Unter dem meteorologischen Datensatz werden gemessene Daten, wie insbesondere Windgeschwindigkeit, Windrichtung, Sonnenscheindauer, Lufttemperatur, Satellitendaten usw. und die für den vorgegebenen Zeitraum auf der Basis der Messdaten errechnete Prognose der Umgebungsbedingungen der Gasspeicher verstanden. Es kann eine Größe, wie beispielsweise die Dauer der Sonneneinstrahlung auf den Gasspeichers während eines Tages gemessen und eine Prognose für den vorgegeben Zeitraum errechnet werden. Im Rahmen der Erfindung können auch verschiedene Größen kombiniert werden, z. B. die Windgeschwindigkeit und die Lufttemperatur.

Die Erfindung beruht auf der Erkenntnis, dass mit Hilfe dieses Verfahrens Biogasanlagen, in denen Biogas erzeugt, gespeichert und einem Gasabnehmer zugeführt wird, so betrieben werden können, dass die Biogasproduktion vorausschauend hinsichtlich der Wetterprognose angepasst werden kann. Die verfügbare Speicherkapazität wird optimal ausgenutzt. Es wird kein Biogas erzeugt, welches nicht speicherbar ist, weil keine Speicherkapazität zur Verfügung steht. Das Verfahren dient zur Verringerung von nicht speicherbaren und somit energetisch nicht nutzbaren Biogasmengen und zur Effizienzsteigerung der Biogasproduktion.

Vorzugsweise wird dem Fermenter die für den vorgegebenen Zeitraum vorgegebene Gesamtmenge an Substrat während des vorgegebenen Zeitraums in unterschiedlichen Mengen pro Zeiteinheit zugeführt, so dass die Erzeugung von Biogas an die Umgebungsbedingungen der Gasspeicher angepasst werden kann.

Ganz besonders vorteilhaft ist ein Verfahren, bei welchem dem Fermenter die für den vorgegebenen Zeitraum vorgegebene Gesamtmenge an Substrat in einer voreingestellte Anzahl von Zufuhren zugeführt wird, wobei die Anzahl der Zufuhren während einer einstellbaren Zeitspanne verringert, insbesondere halbiert, wird und wobei bei den übrigen Zufuhren innerhalb des vorgegebenen Zeitraums die Menge des Substrats vergrößert wird, derart dass die voreingestellte Gesamtmenge an Substrat in dem vorgegebenen Zeitraum nicht verändert wird.

Die übrigen Zufuhren innerhalb des vorgegebenen Zeitraums werden vorzugsweise gleichmäßig vergrößert. Es sind aber auch andere Zufuhrstrategien möglich.

Eine bevorzugte Weiterbildung des Verfahrens, wobei die Anlage mindestens zwei Gasspeicher aufweist, besteht darin, dass in der Regelungseinrichtung aus dem Istwert des Volumens aller Gasspeicher ein Istmittelwert gebildet und in der Regelungseinrichtung hinterlegt wird.

Vorzugsweise wird der Istwert des Gasvolumens oder der Istmittelwert des Gasvolumens um einen ersten Verstellwert in einem Bereich von 0% bis +30% seines Wertes erhöht und/oder um einen zweiten Verstellwert in einem Bereich von -30% bis 0% seines Wertes verringert und der korrigierte Istwert des Gasvolumens und/oder der korrigierte Istmittelwert des Gasvolumens in der Regelungseinrichtung hinterlegt.

Eine bevorzugte Weiterbildung des Verfahrens besteht darin, dass in der Regelungseinrichtung ein Stellsignal zur Erhöhung der Leistung des Gasabnehmers erzeugt wird, wenn der korrigierte Istwert oder der korrigierte Istmittelwert des Gasvolumens den oberen Grenzwert erreicht oder überschreitet und/oder dass in der Regelungseinrichtung ein Stellsignal zur Reduzierung der Leistung des Gasabnehmers erzeugt wird, wenn der korrigierte Istwert oder der korrigierte Istmittelwert des Gasvolumens einen vorgegebenen unteren Grenzwert erreicht oder unterschreitet. Auf diese Weise kann beispielsweise die Soll-Leistung eines Gasabnehmers stufenweise erhöht oder verringert werden. Unter Gasabnehmer wird insbesondere eine Aufbereitungsanlage, ein Blockheizkraftwerk, ein Gaskessel oder eine sonstige Gasverbrauchseinrichtung verstanden. Aufbereitungsanlagen zur Aufbereitung von Biogas zu Bioerdgas, welches in das öffentliche Erdgasnetz eingespeist werden kann, sind insbesondere Anlagen zur Aufbereitung des Biogases mit Hilfe der Druckwechseladsorption, auch "PSA" genannt (Abkürzung von "Pressure Swing Adsorption").

Ein bevorzugtes Verfahren ist gekennzeichnet durch die aufeinander folgenden Schritte:
a) Vergleichen, ob der korrigierte Istwert des Gasvolumen jeden Gasspeichers den oberen Grenzwert erreicht oder überschreitet oder den vorgegebenen unteren Grenzwert erreicht oder unterschreitet
b) Ersetzen der Istwerte des Gasvolumens der einzelnen Gasspeicher durch den korrigierten Istmittelwert, wenn der korrigierte Istwert des Gasvolumens jeden Gasspeichers den oberen Grenzwert nicht erreicht oder überschreitet und wenn der korrigierte Istwert des Gasvolumens jeden Gasspeichers den unteren Grenzwert nicht erreicht oder unterschreitet;
c) Erzeugen eines Stellsignals zur Erhöhung der Leistung des Gasabnehmers, wenn der korrigierte Istmittelwert des Gasvolumens den oberen Grenzwert erreicht oder überschreitet oder wenn der korrigierte Istmittelwert des Gasvolumens den vorgegebenen unteren Grenzwert erreicht oder unterschreitet.

Vorteilhafterweise beträgt der untere Grenzwert 10% der Speicherkapazität des Gasspeichers und der obere Grenzwert 90% der Speicherkapazität des Gasspeichers. Eine bevorzugte Weiterbildung des Verfahrens ist dadurch gekennzeichnet, dass mittels mindestens einer Höhenmesseinrichtung die Höhe der flexiblen Haube des Gasspeichers gemessen wird und dass aus dem Messwert unter Einbeziehung der geometrischen Form der Haube der Istwert des Gasvolumens abgeleitet wird.

Die Höhenmesseinrichtung kann als ein mit einer Flüssigkeit gefüllter Schlauch ausgebildet sein, der sich auf der Haube befindet. Am unteren Ende des Schlauches wird mit einem Druckmessaufnehmer ein zur Höhe der Haube proportionaler Druck gemessen und aus diesem Druckwert unter Einbeziehung der geometrischen Form der Haube der Istwert des Gasvolumens abgeleitet.

Die Erfindung umfasst ferner eine Biogasanlage mit mindestens einem Fermenter, wobei dem Fermenter ein Substrat in einer vorgegebenen Gesamtmenge während eines vorgegebenen Zeitraums zum Vergären zuführt wird, mit mindestens einem Gasspeicher für das Biogas und mit mindestens einen Gasabnehmer, dem Biogas aus dem Gasspeicher zugeführt wird, wobei der Gasspeicher eine flexible Haube aufweist, dadurch gekennzeichnet, dass die Biogasanlage eine Einrichtung zum messtechnischen Erfassen des Istwertes des Gasvolumens im Gasspeicher während des Gärprozesses aufweist, dass eine Regelungseinrichtung dazu ausgebildet ist, das gemessene Istvolumen zu speichern und dass ein oberer Grenzwert und ein unterer Grenzwert für das Gasvolumen in dem Gasspeicher vorgegeben ist und dass vor Beginn des vorgegebenen Zeitraums ein meteorologischer Datensatz in der Regelungseinrichtung hinterlegt ist und dass in Abhängigkeit von dem meteorologischen Datensatz und in Abhängigkeit von der Abweichung des gemessenen Istwertes vom oberen und/oder unteren Grenzwert die Substratzufuhr während des vorgegebenen Zeitraum gesteuert und geregelt wird.

Vorzugsweise bildet der Gasspeicher mit dem Fermenter eine Baueinheit. Der Gasspeicher weist eine flexible Hülle bzw. Haube in Form einer einlagigen elastischen Membran auf. Der Gasspeicher ist somit als Einzelmembran-Gasspeicher ausgebildet. Darunter wird ein Gasspeicher mit nur einer einzigen Membran bzw. einer einlagigen elastischen haubenartigen Hülle bzw. mit einer einlagigen elastischen Behälterabdeckung verstanden.

Eine wesentliche Weiterbildung der Biogasanlage nach der Erfindung besteht darin, dass die Anlage mindestens zwei Gasspeicher aufweist, die kaskadenartig mittels Gasleitungen verbunden sind und dass die Regelungseinrichtung dazu ausgebildet ist, aus dem Istwert des Gasvolumens aller Gasspeicher einen Istmittelwert zu bilden und um den Istwert oder den Istmittelwert um einen ersten Verstellwert in einem Bereich von 0% bis +30% seines Wertes zu erhöhen und/oder um einen zweiten Verstellwert in einem Bereich von -30% bis 0% seines Wertes zu verringern und dass der korrigierte Istwert oder der korrigierte Istmittelwert in der Regelungseinrichtung hinterlegbar ist.

In Weiterbildung der Erfindung ist die Biogasanlage dadurch gekennzeichnet, dass die Regelungseinrichtung dazu ausgebildet ist, ein Stellsignal zur Erhöhung der Leistung des Gasabnehmers zu erzeugen, wenn der korrigierte Istwert und/oder der korrigierte Istmittelwert des Gasvolumens den oberen Grenzwert erreicht oder überschreitet und/ oder ein Stellsignal zur Reduzierung der Leistung des Gasabnehmers zu erzeugen, wenn der korrigierte Istwert und/oder der korrigierte Istmittelwert des Gasvolumens einen vorgegebenen unteren Grenzwert erreicht oder unterschreitet.

Im Rahmen der Erfindung kann der Gasabnehmer als Aufbereitungsanlage ausgebildet sein, in der insbesondere das Kohlendioxid aus dem Biogas abgetrennt wird. Derartige Aufbereitungsanlagen sind insbesondere Anlagen zur Aufbereitung des Biogases mit Hilfe der Druckwechseladsorption, auch "PSA" genannt (Abkürzung von "Pressure Swing Adsorption"). Alternativ kann der Gasabnehmer als Blockheizkraftwerk oder Gaskessel oder als sonstige Gasverbrauchseinrichtung ausgebildet sein.

Die Erfindung wird im Folgenden anhand eines bevorzugten Ausführungsbeispiels einer erfindungsgemäßen Anlage zum Erzeugen von Biogas im Zusammenhang mit den Zeichnungen näher erläutert.

Die Zeichnungen zeigen in:
Fig. 1 eine schematische Darstellung eine Biogasanlage nach der Erfindung;
Fig. 2 eine schematische Darstellung einer Ausführungsform einer Regelung nach der Erfindung.

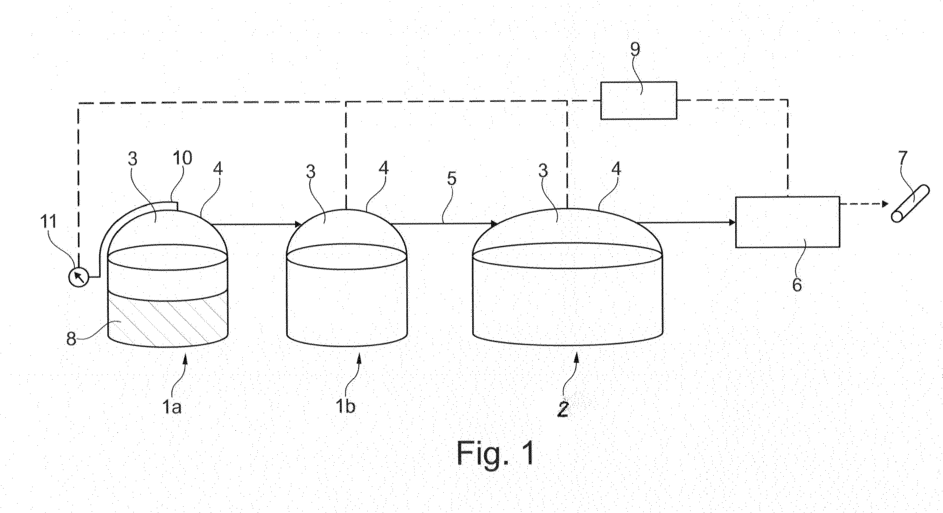

Fig. 1 zeigt schematisch eine Biogasanlage. Zwei Fermentern 1a, 1b und ein weiterer Fermenter in Form eines Gärrestlagers 2 sind mit Gasspeichern 3 versehen, in denen das während des Gärprozesses entstehende Biogas gesammelt und gespeichert wird. Die Gasspeicher 3 bildet mit den Fermenter 1a, 1b, 2 jeweils eine Baueinheit. Die Gasspeicher 3 sind jeweils mit einer flexiblen Haube 4 versehen, die als einlagige Haube bzw. als Einzelmembran ausgeführt ist, d. h. dass das im Speicher befindliche Gas nur durch eine einlagige elastische haubenartige Hülle von der Umgebung getrennt ist. Das Biogas wird daher von den Umweltbedingungen, insbesondere der Sonneneinstrahlung in der Dichte in einem relativ großen Maß beeinflusst. Durch starke Sonneneinstrahlung dehnt sich das gespeicherte Gasvolumen unter der Haube 4 aus und verringert somit die Speicherkapazität der Gashaube bzw. Haube 4. Dies wirkt sich besonders bei einlagigen Gasspeichern aus.

Die Fermenter 1a und 1b und das Gärrestelager 2 sind teilweise mit Substrat 8 bzw. Biomasse bzw. mit Gärresten gefüllt. Dem Fermenter 1a wird das Substrat in einer vorgegebenen Gesamtmenge während eines vorgegebenen Zeitraums, hier 24 Stunden bzw. ein Tag zum Vergären zugeführt.

Alle Gasspeicher 3 der Biogasanlage sind über eine Biogasleitung 5 miteinander verbunden. Das Biogas wird einer Aufbereitungsanlage 6 in Form einer PSA-Anlage zugeführt und durch Entfernung von insbesondere CO₂ zu Bioerdgas umgewandelt, welches in ein öffentliches Erdgasversorgungsnetz 7 eingeleitet wird.

In einer Regelungseinrichtung 9 sind für jeden Gasspeicher 3 obere und untere Grenzwerte für das Gasvolumen hinterlegt. Der Istwert des Gasvolumens aller Gasspeicher wird messtechnisch erfasst. Vor Beginn des vorgegebenen Zeitraums von 24 Stunden wird ein meteorologischer Datensatz, insbesondere gemessene Daten wie Windgeschwindigkeit und/oder Windrichtung, Sonnenscheindauer, Lufttemperatur und die für den vorgegebenen Zeitraum auf der Basis der Messdaten errechnete Prognose der Umgebungsbedingungen der Gasspeicher in der Regelungseinrichtung 9 hinterlegt und in Abhängigkeit von dem meteorologischen Datensatz und von der Abweichung des Istmittelwertes von dem Sollwert des Gasvolumens ein Steuersignal zur Steuerung der Substratzufuhr während des vorgegebenen Zeitraums gebildet.

Dem Fermenter 1a wird die für den Zeitraum von 24 Stunden vorgegebene Gesamtmenge an Substrat in einer voreingestellten Anzahl von Zufuhren zugeführt. Nach der Erfindung wird die Anzahl der Zufuhren in Abhängigkeit von dem meteorologischen Datensatz während einer einstellbaren Zeitspanne verringert, insbesondere halbiert. Bei den übrigen Zufuhren innerhalb der 24 Stunden wird die Menge des Substrats pro Einzelzufuhr gleichmäßig vergrößert, so dass die voreingestellte Gesamtmenge an Substrat in dem vorgegebenen Zeitraum nicht verändert wird.

Die Steuerung für die Fermenter bietet also die Möglichkeit, in Abhängigkeit von dem meteorologischen Datensatz automatisch eine einstellbare Anzahl von Substratzufuhren bzw. Fütterungen herauszunehmen. Die zeitweise nicht zugeführte Substratmenge wird im Anschluss auf die für den Tag noch anstehenden Fütterungen gleichmäßig verteilt.

Mit Hilfe des erfindungsgemäßen Verfahrens kann die Biogasanlage so betrieben werden, dass die Rohbiogasproduktion vorausschauend hinsichtlich der Wetterprognose angepasst werden kann. Damit wird vermieden, dass Biogas abgefackelt werden muss.

Zur messtechnischen Erfassung des Gasvolumens in dem Gasspeicher 3 befindet sich auf jedem Gasspeicher ein, nur bei dem Gasspeicher 3 des Fermenters 1a dargestellter, mit einer Flüssigkeit gefüllter Schlauch 10, an dessen unteren Ende ein Druckmessaufnehmer 11 angeordnet ist. Es wird ein zur Höhe der Haube 4 proportionaler Druck gemessen und aus dem Messwert unter Einbeziehung der geometrischen Form der Haube 4 in der Regelungseinrichtung 9 der Istwert des Gasvolumens abgeleitet. Zur Verbesserung der Messgenauigkeit können sich auf jedem Gasspeicher 3 beispielsweise drei derartiger Messeinrichtungen befinden.

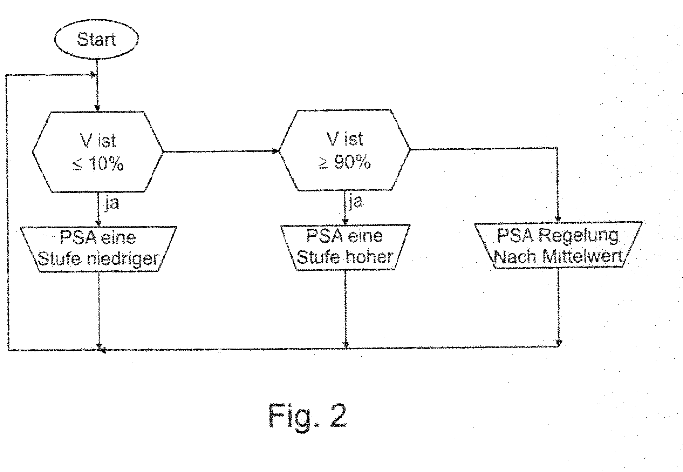

In Fig. 2 ist ein Regelkreis dargestellt. In jedem Gasspeicher wird das Istvolumen gemessen. Zunächst wird verglichen, ob der Istwert Vᵢₛₜ des Gasvolumens in jedem Gasspeicher gleich einem vorgegebenen unteren Grenzwert in Höhe von 10% der Speicherkapazität des Gasspeichers ist oder diesen unterschreitet. Wenn einer der gemessenen Istwerte Vᵢₛₜ des Gasvolumens den unteren Grenzwert erreicht oder unterschreitet, wird ein Stellsignal erzeugt, mit dem die Sollleistung der Aufbereitungsanlage 6 in Form einer PSA-Anlage (Pressure Swing Adsorption) zur Aufbereitung des Biogases zu Bioerdgas um eine Stufe verringert wird.

Ferner wird verglichen, ob der Istwert des Gasvolumens in jedem Gasspeichers Vᵢₛₜ gleich einem vorgegebenen oberen Grenzwert in Höhe von 90% der Speicherkapazität des Gasspeichers ist oder diesen Grenzwert überschreitet. Wenn einer der Istwerte den vorgegebenen oberen Grenzwert erreicht oder überschreitet wird in der Regelungseinrichtung ein Steuersignal erzeugt, mit dem die Leistung der PSA-Aufbereitungsanlage 6 erhöht wird. Die Sollleistung der PSA-Aufbereitungsanlage 6 kann beispielsweise um eine Stufe erhöht werden.

Wenn keiner der gemessenen Istwerte des Gasvolumens den oberen und unteren Grenzwert erreicht, werden die Istwerte des Gasvolumens der einzelnen Gasspeicher in der Regelungseinrichtung durch einen Istmittelwert ersetzt.

Der Istmittelwert des Gasvolumens kann um einen ersten Verstellwert in einem Bereich von 0% bis +30% seines Wertes erhöht und um einen zweiten Verstellwert in einem Bereich von -30% bis 0% seines Wertes verringert werden. Ein sich rechnerisch ergebender Mittelwert von beispielsweise 70% kann bis zu 91% durch Verstellen erhöht bzw. bis 49% verringert werden. Zusätzlich kann jeder einzelne Messwert des Gasvolumens verstellt bzw. mit einem Offset belegt werden. Durch diese Offset-Einstellmöglichkeit wird der Istwert der Gasvolumens verschoben und dadurch eine Regelungskorrektur erreicht. Was wiederum bewirkt, dass schneller ein Stellsignal zur Verringerung oder Erhöhung der Soll-Leistung des Gasabnehmers, beispielsweise eine Aufbereitungsanlage, erzeugt wird.

Zur Absicherung der Biogasanlage ist diese abhängig von der Größe mit einer oder mehreren nicht dargestellten Rohbiogasfackeln ausgerüstet. Zur zusätzlichen Absicherung ist jeder Gasspeicher 3 mit nicht dargestellten mechanisch arbeitenden Überdrucksicherungen ausgestattet.

Falls eine Anlage mehr als einen Erzeugungsstrang aufweist, kann ein hydraulischer Abgleich zwischen den Biogaserzeugungssträngen mittels einer Gasklappe vorgenommen werden, die sich in einer Gasleitung befindet, die beide Erzeugungsstränge verbindet.

Versuche haben gezeigt, dass mit dem erfindungsgemäßen Verfahren die Gasmengen, die bisher über die Fackeln energetisch ungenutzt geführt wurden, auf 0,1% der eingespeisten Gasmenge reduziert werden konnten.

**Bezugszeichenliste**

| | |
|---|---|
| 1a, 1b | Fermenter |
| 2 | Fermenter / Gärrestelager |
| 3 | Gasspeicher |
| 4 | flexible Haube |
| 5 | Biogasleitung |
| 6 | Gasabnehmer / Aufbereitungsanlage |
| 7 | öffentliches Gasversorgungsnetz |
| 8 | Substrat |
| 9 | Regelungseinrichtung |
| 10 | Schlauch |
| 11 | Druckmessaufnehmer |
| | |
| PSA | Pressure Swing Adsorption Aufbereitungsanlage |

## Patentansprüche

1. Verfahren zum Betreiben einer Biogasanlage, die mindestens einen Fermenter (1a, 1b, 2), mindestens einen Gasspeicher (3) für das Biogas und mindestens einen Gasabnehmer (6) aufweist, wobei dem Fermenter ein Substrat (8) in einer vorgegebenen Gesamtmenge während eines vorgegebenen Zeitraums zum Vergären zugeführt wird und dem Gasabnehmer (6) Biogas aus dem Gasspeicher (3) zugeleitet wird, **dadurch gekennzeichnet,**
**dass** in einer Regelungseinrichtung (9) ein vorgegebener oberer Grenzwert und ein vorgegebener unterer Grenzwert für das Gasvolumen in dem Gasspeicher hinterlegt sind, dass während des Gärprozesses der Istwert des Gasvolumens in dem Gasspeicher (3) messtechnisch erfasst und in der Regelungseinrichtung (9) gespeichert wird, dass vor Beginn des vorgegebenen Zeitraums ein meteorologischer Datensatz in der Regelungseinrichtung (9) hinterlegt wird und dass in Abhängigkeit von dem meteorologischen Datensatz und in Abhängigkeit von der Abweichung des gemessenen Istwertes vom oberen Grenzwert und/oder unteren Grenzwert die Substratzufuhr während des vorgegebenen Zeitraums gesteuert oder geregelt wird.

2. Verfahren nach Anspruch 1,
**dadurch gekennzeichnet, dass** dem Fermenter die für den vorgegebenen Zeitraum vorgegebene Gesamtmenge an Substrat (8) während des vorgegebenen Zeitraums in unterschiedlichen Mengen pro Zeiteinheit zugeführt wird.

3. Verfahren nach wenigstens einem der vorangegangenen Ansprüche,
**dadurch gekennzeichnet, dass** dem Fermenter die für den vorgegebenen Zeitraum vorgegebene Gesamtmenge an Substrat (8) in einer voreingestellten Anzahl von Zufuhren zugeführt wird und dass die Anzahl der Zufuhren während einer steuerbaren Zeitspanne verringert, insbesondere halbiert wird und dass bei den übrigen Zufuhren innerhalb des vorgegebenen Zeitraums die Menge des Substrats vergrößert wird, derart dass die voreingestellte Gesamtmenge an Substrat in dem vorgegebenen Zeitraum nicht verändert wird.

4. Verfahren nach wenigstens einem der vorangegangenen Ansprüche,
wobei die Biogasanlage mindestens zwei Gasspeicher (3) aufweist, **dadurch gekennzeichnet, dass** in der Regelungseinrichtung (9) aus dem Istwert des Volumens aller Gasspeicher (3) ein Istmittelwert gebildet und in der Regelungseinrichtung (9) hinterlegt wird.

5. Verfahren nach wenigstens einem der vorangegangenen Ansprüche,
**dadurch gekennzeichnet, dass** der Istwert des Gasvolumens eines Gasspeichers (3) oder der Istmittelwert um einen ersten Verstellwert in einem Bereich von 0% bis +30% seines Wertes erhöht wird und/oder um einen zweiten Verstellwert in einem Bereich von -30% bis 0% seines Wertes verringert wird und dass der korrigierte Istwert und/oder der korrigierte Istmittelwert in der Regelungseinrichtung (9) hinterlegt wird bzw. werden.

6. Verfahren nach wenigstens einem der vorangegangenen Ansprüche,
**dadurch gekennzeichnet, dass** in der Regelungseinrichtung (9) ein Stellsignal zur Erhöhung der Leistung des Gasabnehmers (6) erzeugt wird, wenn der korrigierte Istwert oder der korrigierte Istmittelwert des Gasvolumens den oberen Grenzwert erreicht oder überschreitet und/oder dass in der Regelungseinrichtung (9) ein Stellsignal zur Reduzierung der Leistung des Gasabnehmers (6) erzeugt wird, wenn der korrigierte Istwert oder der korrigierte Istmittelwert des Gasvolumens einen vorgegebenen unteren Grenzwert erreicht oder unterschreitet.

7. Verfahren nach wenigstens einem der vorangegangenen Ansprüche,
**gekennzeichnet durch** die aufeinander folgenden Schritte:
a) Vergleichen, ob der korrigierte Istwert des Gasvolumens jeden Gasspeichers (3) den oberen Grenzwert erreicht oder überschreitet oder den vorgegebenen unteren Grenzwert erreicht oder unterschreitet;
b) Ersetzen der Istwerte des Gasvolumens der einzelnen Gasspeicher (3) **durch** den korrigierten Istmittelwert, wenn der korrigierte Istwert des Gasvolumens jeden Gasspeichers (3) den oberen Grenzwert nicht erreicht oder überschreitet und wenn der korrigierte Istwert jedes Gasspeichers den untere Grenzwert nicht erreicht oder unterschreitet;
c) Erzeugen eines Stellsignals zur Erhöhung der Leistung des Gasabnehmers, wenn der korrigierte Istmittelwert des Gasvolumens den oberen Grenzwert erreicht oder überschreitet oder wenn der korrigierte Istmittelwert des Gasvolumens den vorgegebenen unteren Grenzwert erreicht oder unterschreitet.

8. Verfahren nach wenigstens einem der vorangegangenen Ansprüche,
**dadurch gekennzeichnet, dass** der untere Grenzwert 10% der Speicherkapazität des Gasspeichers und der obere Grenzwert 90% der Speicherkapazität des Gasspeichers beträgt.

9. Verfahren nach wenigstens einem der vorangegangenen Ansprüche,
**dadurch gekennzeichnet, dass** mittels mindestens einer Höhenmesseinrichtung die Höhe der flexiblen Haube (4) des Gasspeichers (3) gemessen wird und dass aus dem Messwert unter Einbeziehung der geometrischen Form der Haube (4) der Istwert des Gasvolumens abgeleitet wird.

10. Biogasanlage, mit mindestens einem Fermenter (1a, 1b, 2), wobei dem Fermenter ein Substrat in einer vorgegebenen Gesamtmenge während eines vorgegebenen Zeitraums zum Vergären zuführt wird, mit mindestens einem Gasspeicher (3) für das Biogas und mit mindestens einen Gasabnehmer (6), dem Biogas aus dem Gasspeicher (3) zugeführt wird, wobei der Gasspeicher (3) eine flexible Haube (4) aufweist,
**dadurch gekennzeichnet,**
**dass** die Biogasanlage eine Einrichtung (10, 11) zum messtechnischen Erfassen des Istwertes des Gasvolumens im Gasspeicher während des Gärprozesseses aufweist, dass eine Regelungseinrichtung (9) dazu ausgebildet ist, dass das gemessene Istvolumen gespeichert wird und dass ein oberer Grenzwert und ein unterer Grenzwert für das Gasvolumen in dem Gasspeicher (3) vorgegeben ist und dass vor Beginn des vorgegebenen Zeitraums ein meteorologischer Datensatz in der Regelungseinrichtung (9) hinterlegt ist und dass in Abhängigkeit von dem meteorologischen Datensatz und in Abhängigkeit von der Abweichung des gemessenen Istwertes vom oberen und/oder unteren Grenzwert die Substratzufuhr während des vorgegebenen Zeitraums gesteuert oder geregelt wird.

11. Biogasanlage nach Anspruch 10,
**dadurch gekennzeichnet, dass** der Gasspeicher (3) mit dem Fermenter (1a, 1b, 2) eine Baueinheit bildet und dass der Gasspeicher (3) eine flexible Haube (4) in Form einer einlagigen Membran aufweist.

12. Biogasanlage nach wenigstens einem der vorangegangenen Ansprüche 10 bis 11, **dadurch gekennzeichnet, dass** die Biogasanlage mindestens zwei Gasspeicher (3) aufweist, die kaskadenartig mittels Gasleitungen (5) verbunden sind und dass die Regelungseinrichtung (9) dazu ausgebildet ist, aus dem Istwert des Gasvolumens aller Gasspeicher (3) einen Istmittelwert zu bilden, um den Istwert oder den Istmittelwert um einen ersten Verstellwert in einem Bereich von 0% bis +30% seines Wertes zu erhöhen und/oder um einen zweiten Verstellwert in einem Bereich von -30% bis 0% seines Wertes zu verringern und dass der korrigierte Istwert oder der korrigierte Istmittelwert in der Regelungseinrichtung (9) hinterlegbar ist.

13. Biogasanlage nach wenigstens einem der vorangegangenen Ansprüche 10 bis 12, **dadurch gekennzeichnet, dass** die Regelungseinrichtung (9) dazu ausgebildet ist, ein Stellsignal zur Erhöhung der Leistung des Gasabnehmers (6) zu erzeugen, wenn der korrigierte Istwert und/oder der korrigierte Istmittelwert des Gasvolumens den oberen Grenzwert erreicht oder überschreitet und/oder ein Stellsignal zur Reduzierung der Leistung des Gasabnehmers (6) zu erzeugen, wenn der korrigierte Istwert und/oder der korrigierte Istmittelwert des Gasvolumens einen vorgegebenen unteren Grenzwert erreicht oder unterschreitet.

14. Biogasanlage nach wenigstens einem der vorangegangenen Ansprüche 10 bis 13, **dadurch gekennzeichnet, dass** der Gasabnehmer (6) als Aufbereitungsanlage, in der insbesondere das Kohlendioxid aus dem Rohbiogas abgetrennt wird oder dass der Gasabnehmer (6) als Blockheizkraftwerk oder Gaskessel ausgebildet ist.

## Claims

1. Method for operating a biogas plant having at least one fermenter (1a, 1b, 2), at least one gas holder (3) for the biogas and at least one gas consumer (6), with a substrate (8) being fed to the fermenter in a predetermined total quantity during a predetermined period of time for fermentation and biogas from the gas holder (3) being fed to the gas consumer (6),
**characterised in that** a predetermined upper limit value and a predetermined lower limit value for the gas volume in the gas holder are stored in a control device (9), **in that** the actual value of the gas volume in the gas holder (3) is measured during the fermentation process and stored in the control device (9), **in that** a meteorological data set is stored in the control device (9) before the start of the predetermined time period and **in that** substrate feeding is controlled in an open or closed-loop manner during the predetermined time period as a function of the meteorological data set and as a function of the deviation of the measured actual value from the upper limit value and/or lower limit value.

2. Method according to claim 1,
**characterised in that** the total quantity of substrate (8) predetermined for the predetermined period of time is fed to the fermenter in different quantities per unit of time during the predetermined period of time.

3. Method according to at least one of the preceding claims,
**characterised in that** the total quantity of substrate (8) predetermined for the predetermined period of time is fed to the fermenter in a pre-set number of feeds and that the number of feeds is reduced, in particular halved, during a controllable period of time and that for the remaining feeds within the predetermined period of time the quantity of substrate is increased in such a way that the pre-set total quantity of substrate is not changed in the predetermined period of time.

4. Method according to at least one of the preceding claims,
wherein the biogas plant has at least two gas holders (3), **characterised in that** in the control device (9) an actual mean value is formed from the actual value of the volume of all gas holders (3) and is stored in the control device (9).

5. Method according to at least one of the preceding claims,
**characterised in that** the actual value of the gas volume of a gas holder (3) or the actual mean value is increased by a first adjustment value in a range of 0% to +30% of its value and/or is reduced by a second adjustment value in a range of -30% to 0% of its value and that the corrected actual value and/or the corrected actual mean value is/are stored in the control device (9).

6. Method according to at least one of the preceding claims,
**characterised in that** an actuating signal for increasing the output of the gas consumer (6) is generated in the control device (9) when the corrected actual value or the corrected actual mean value of the gas volume reaches or exceeds the upper limit value and/or **in that** an actuating signal for reducing the output of the gas consumer (6) is generated in the control device (9) when the corrected actual value or the corrected actual mean value of the gas volume reaches or falls below a predetermined lower limit value.

7. Method according to at least one of the preceding claims,
**characterised by** the successive steps:
a) comparing whether the corrected actual value of the gas volume of each gas holder (3) reaches or exceeds the upper limit value or reaches or falls below the predetermined lower limit value;
b) replacing the actual values of the gas volume of the individual gas holders (3) by the corrected actual mean value when the corrected actual value of the gas volume of each gas holder (3) does not reach or exceed the upper limit value and if the corrected actual value of each gas accumulator does not reach or fall below the lower limit value;
c) generating an actuating signal to increase the output of the gas consumer when the corrected actual mean value of the gas volume reaches or exceeds the upper limit value or when the corrected actual mean value of the gas volume reaches or falls below the predetermined lower limit value.

8. Method according to at least one of the preceding claims,
**characterised in that** the lower limit value is 10% of the storage capacity of the gas holder and the upper limit value is 90% of the storage capacity of the gas holder.

9. Method according to at least one of the preceding claims,
**characterised in that** the height of the flexible hood (4) of the gas holder (3) is measured by means of at least one height measuring device and **in that** the actual value of the gas volume is derived from the measured value, taking into account the geometric shape of the hood (4).

10. Biogas plant having at least one fermenter (1a, 1b, 2), with a substrate being fed to the fermenter in a predetermined total quantity during a predetermined period for fermentation, having at least one gas holder (3) for the biogas and having at least one gas consumer (6) to which biogas is fed from the gas holder (3), with the gas holder (3) having a flexible hood (4),
**characterised in that**
the biogas plant has a device (10, 11) for the metrological detection of the actual value of the gas volume in the gas holder during the fermentation process, **in that** a control device (9) is designed in such a way that the measured actual volume is stored and so that an upper limit value and a lower limit value for the gas volume in the gas holder (3) are predetermined, and **in that** a meteorological data set is stored in the control device (9) before the start of the predetermined time period, and **in that** substrate feeding is controlled in an open or closed loop manner during the predetermined time period as a function of the meteorological data set and as a function of the deviation of the measured actual value from the upper and/or lower limit value.

11. Biogas plant according to claim 10,
**characterised in that** the gas holder (3) forms a structural unit with the fermenter (1a, 1b, 2) and that the gas holder (3) exhibits a flexible hood (4) in the form of a single-layer membrane.

12. Biogas plant according to at least one of the preceding claims 10 to 11, **characterised in** the biogas plant has at least two gas holders (3) which are connected in cascade by means of gas piping (5) and **in that** the control device (9) is designed to form an actual mean value from the actual value of the gas volume of all gas holders (3) in order to increase the actual value or the actual mean value by a first adjustment value in a range of 0% to +30% of its value and/or to reduce it by a second adjustment value in a range of -30% to 0% of its value, and **in that** the corrected actual value or the corrected actual mean value can be stored in the control device (9).

13. Biogas plant according to at least one of the preceding claims 10 to 12, **characterised in that** the control device (9) is designed to generate an actuating signal for increasing the output of the gas consumer (6) when the corrected actual value and/or the corrected actual mean value of the gas volume reaches or exceeds the upper limit value and/or to generate an actuating signal for reducing the output of the gas consumer (6) when the corrected actual value and/or the corrected actual mean value of the gas volume reaches or falls below a predetermined lower limit value.

14. Biogas plant according to at least one of the preceding claims 10 to 13, **characterised in that** the gas consumer (6) is designed as a treatment plant in which in particular the carbon dioxide is separated from the raw biogas, or **in that** the gas consumer (6) is designed as a small-scale combined heat and power plant or a gas boiler.

## Revendications

1. Procédé de fonctionnement d'une installation de production de biogaz qui présente au moins un fermenteur (1a, 1b, 2), au moins un réservoir de gaz (3) pour le biogaz et au moins un consommateur de gaz (6), un substrat (8) étant amené au fermenteur en une quantité totale prédéfinie pendant une période prédéfinie pour sa fermentation et le biogaz étant amené au consommateur de gaz (6) à partir du réservoir de gaz (3), **caractérisé en ce**
**qu'**une valeur limite supérieure et une valeur limite inférieure prédéfinies pour le volume de gaz dans le réservoir de gaz sont enregistrées dans un dispositif de régulation (9), que la valeur réelle du volume de gaz dans le réservoir de gaz (3) est mesurée pendant le processus de fermentation et stockée dans le dispositif de régulation (9), qu'un jeu de données météorologiques est enregistré dans le dispositif de régulation (9) avant le début de la période prédéfinie et que l'amenée de substrat est commandée ou régulée pendant la période prédéfinie en fonction du jeu de données météorologiques et en fonction de l'écart entre la valeur réelle mesurée et la valeur limite supérieure et/ou la valeur limite inférieure.

2. Procédé selon la revendication 1,
**caractérisé en ce que** la quantité totale prédéfinie de substrat (8) pour la période prédéfinie est amenée au fermenteur en différentes quantités par unité de temps pendant la période prédéfinie.

3. Procédé selon au moins l'une des revendications précédentes,
**caractérisé en ce que** la quantité totale prédéfinie de substrat (8) pour la période prédéfinie est amenée au fermenteur en un nombre d'amenées prédéfini, que le nombre d'amenées est réduit, en particulier divisé par deux, pendant un intervalle de temps commandable et que, pour les amenées restantes dans la période prédéfinie, la quantité de substrat est augmentée de telle manière que la quantité totale prédéfinie de substrat ne soit pas modifiée dans la période prédéfinie.

4. Procédé selon au moins l'une des revendications précédentes,
dans lequel l'installation de production de biogaz présente au moins deux réservoirs de gaz (3), **caractérisé en ce qu'**une valeur moyenne réelle est formée dans le dispositif de régulation (9) à partir de la valeur réelle du volume de tous les réservoirs de gaz (3) et enregistrée dans le dispositif de régulation (9).

5. Procédé selon au moins l'une des revendications précédentes,
**caractérisé en ce que** la valeur réelle du volume de gaz d'un réservoir de gaz (3) ou la valeur moyenne réelle est augmentée d'une première valeur de réglage dans une plage de 0 % à +30 % de sa valeur et/ou est diminuée d'une deuxième valeur de réglage dans une plage de -30 % à 0 % de sa valeur et que la valeur réelle corrigée et/ou la valeur moyenne réelle corrigée est/sont enregistrée(s) dans le dispositif de régulation (9).

6. Procédé selon au moins l'une des revendications précédentes,
**caractérisé en ce qu'**un signal de réglage pour augmenter la puissance du consommateur de gaz (6) est généré dans le dispositif de régulation (9) lorsque la valeur réelle corrigée ou la valeur moyenne réelle corrigée du volume de gaz atteint ou est supérieure à la valeur limite supérieure et/ou qu'un signal de réglage pour réduire la puissance du consommateur de gaz (6) est généré dans le dispositif de régulation (9) lorsque la valeur réelle corrigée ou la valeur moyenne réelle corrigée du volume de gaz atteint ou est inférieure à une valeur limite inférieure prédéfinie.

7. Procédé selon au moins l'une des revendications précédentes,
**caractérisé par** les étapes successives consistant à :
a) comparer si la valeur réelle corrigée du volume de gaz de chaque réservoir de gaz (3) atteint ou est supérieure à la valeur limite supérieure ou atteint ou est inférieure à la valeur limite inférieure prédéfinie ;
b) remplacer les valeurs réelles du volume de gaz des différents réservoirs de gaz (3) par la valeur moyenne réelle corrigée si la valeur réelle corrigée du volume de gaz de chaque réservoir de gaz (3) n'atteint pas ou n'est pas supérieure à la valeur limite supérieure et si la valeur réelle corrigée de chaque réservoir à gaz n'atteint pas ou n'est pas inférieure à la valeur limite inférieure ;
c) générer un signal de réglage pour augmenter la puissance du consommateur de gaz si la valeur moyenne réelle corrigée du volume de gaz atteint ou est supérieure à la valeur limite supérieure ou si la valeur moyenne réelle corrigée du volume de gaz atteint ou est inférieure à la valeur limite inférieure prédéfinie.

8. Procédé selon au moins l'une des revendications précédentes,
**caractérisé en ce que** la valeur limite inférieure est de 10 % de la capacité de stockage du réservoir de gaz et la limite supérieure est de 90 % de la capacité de stockage du réservoir de gaz.

9. Procédé selon au moins l'une des revendications précédentes,
**caractérisé en ce que** la hauteur de la calotte flexible (4) du réservoir de gaz (3) est mesurée au moyen d'au moins un dispositif de mesure de hauteur et que la valeur réelle du volume de gaz est dérivée de la valeur mesurée en tenant compte de la forme géométrique de la calotte (4).

10. Installation de production de biogaz, présentant au moins un fermenteur (1a, 1b, 2), dans laquelle un substrat est amené au fermenteur en une quantité totale prédéfinie pendant une période prédéfinie pour sa fermentation, au moins un réservoir de gaz (3) pour le biogaz et au moins un consommateur de gaz (6) auquel le biogaz est amené à partir du réservoir de gaz (3), le réservoir de gaz (3) présentant une calotte flexible (4),
**caractérisée en ce**
**que** l'installation de production de biogaz présente un dispositif (10, 11) pour mesurer la valeur réelle du volume de gaz dans le réservoir de gaz pendant le processus de fermentation, qu'un dispositif de régulation (9) est conçu de telle sorte que le volume réel mesuré est stocké, qu'une valeur limite supérieure et une valeur limite inférieure pour le volume de gaz dans le réservoir de gaz (3) sont prédéfinies, qu'un jeu de données météorologiques est enregistré dans le dispositif de régulation (9) avant le début de la période prédéfinie et que l'amenée de substrat pendant la période prédéfinie est commandée ou régulée en fonction du jeu de données météorologiques et en fonction de l'écart entre la valeur réelle mesurée et la valeur limite supérieure et/ou inférieure.

11. Installation de production de biogaz selon la revendication 10,
**caractérisée en ce que** le réservoir de gaz (3) forme une unité structurale avec le fermenteur (1a, 1b, 2) et que le réservoir de gaz (3) présente une calotte flexible (4) sous la forme d'une membrane monocouche.

12. Installation de production de biogaz selon au moins l'une des revendications 10 à 11 précédentes,
**caractérisée en ce que** l'installation de production de biogaz présente au moins deux réservoirs de gaz (3) qui sont reliés en cascade au moyen de conduites de gaz (5) et que le dispositif de régulation (9) est conçu pour former une valeur moyenne réelle à partir de la valeur réelle du volume de gaz de tous les réservoirs de gaz (3), afin d'augmenter la valeur réelle ou la valeur moyenne réelle d'une première valeur de réglage dans une plage de 0 % à +30 % de sa valeur et/ou de la diminuer d'une deuxième valeur de réglage dans une plage de -30 % à 0 % de sa valeur et que la valeur réelle corrigée ou la valeur moyenne réelle corrigée peut être enregistrée dans le dispositif de régulation (9).

13. Installation de production de biogaz selon au moins l'une des revendications 10 à 12 précédentes,
**caractérisée en ce que** le dispositif de régulation (9) est conçu pour générer un signal de réglage pour augmenter la puissance du consommateur de gaz (6) lorsque la valeur réelle corrigée et/ou la valeur moyenne réelle corrigée du volume de gaz atteint ou est supérieure à la valeur limite supérieure et/ou pour générer un signal de réglage pour réduire la puissance du consommateur de gaz (6) lorsque la valeur réelle corrigée et/ou la valeur moyenne réelle corrigée du volume de gaz atteint ou est inférieure à une valeur limite inférieure prédéfinie.

14. Installation de production de biogaz selon au moins l'une des revendications 10 à 13 précédentes,
**caractérisée en ce que** le consommateur de gaz (6) est conçu comme une installation de traitement dans laquelle, en particulier, le dioxyde de carbone est séparé du biogaz brut ou que le consommateur de gaz (6) est conçu comme une unité de production combinée de chaleur et d'électricité ou une chaudière à gaz.
